# EUROPEAN PATENT SPECIFICATION

(11) **EP 0 371 399 B1**
(45) Date of publication and mention of the grant of the patent: **04.08.1993**
(21) Application number: 89121665.7
(22) Date of filing: 23.11.1989
(51) Int. Cl.: C07C 31/40, C07C 29/124

(54) **Process for preparing fluoroalcohols**
Verfahren zur Herstellung von fluorierten Alkoholen
Procédé de préparation d'alcools fluorés

(30) Priority: 24.11.1988 JP 297000/88; 25.11.1988 JP 298818/88
(43) Date of publication of application: 06.06.1990
(73) Proprietor: DAIKIN INDUSTRIES, LIMITED, Osaka-shi, Osaka-fu (JP)
(72) Inventor: Yoshida, Tutomu, Ibaraki-shi Osaka-fu (JP); Tanaka, Kunitada, Osaka-shi Osaka-fu (JP); Chiba, Yasumichi, Settsu-shi Osaka-fu (JP); Furutaka, Yasuhisa, Takatsuki-shi Osaka-fu (JP); Homoto, Yukio, Katano-shi Osaka-fu (JP)
(74) Representative: Barz, Peter, Dr.

(56) References cited:
- DE-A- 3 016 571
- FR-A- 2 180 113

## Description

The present invention relates to processes for preparing a fluoroalcohol.

Fluoroalcohols are useful as an intermediate for water and oil repellent agent, surfactant, releasing agent or the like.

As processes for preparing a fluoroalcohol, the following methods are conventionally known.
① A method of contacting a starting fluoroalkyl iodide or bromide compound with fuming sulfuric acid or chlorosulfuric acid to prepare a sulfate and subsequently subjecting the sulfate to hydrolysis (JP-B-40-19085 and JP-B-58-39135),
② A method of contacting the same starting compound as above with dimethyl formamide and water (JP-B-52-8807),
③ A method of subjecting the same starting compound to hydrolysis in an aqueous solution of a monoxidative oxyacid or hydrogen iodide at a pH of up to 2 (DE 2318677 A),
④ A method of contacting the same starting compound with water in an organic solvent in the presence of a heavy metal ion catalyst (JP-A-63-22040), etc.

However, in the method ①, fuming sulfuric acid and chlorosulfuric acid are strong in corrosion activity and a lot of by-products are formed such as dialkylsulfates and chlorides which are hardly subjected to hydrolysis. The method ② forms dimethylamine and formic acid as by-products, hence problem in recovery thereof. Further, a lot of compounds having terminal double bond are formed. In the method ③, it is difficult to choose a material durable to HI at a strong acidity of up to pH 2, at high temperature more than 200°C and at high pressure. The method ④ has problems in toxicity of heavy metal, pollution derived therefrom and recovery of the metal.

An object of the present invention is to provide a process for preparing a fluoroalcohol which is safe and high in yield without use of dangerous chemicals and heavy metals.

The above and other objects of the invention will become apparent from the following description.

As a first embodiment, the present invention provides a process for preparing a fluoroalcohol of the formula

Rf(CH₂CH₂OH)n (1)

which is a single-step process and comprises contacting a betaine compound and water in the presence of an organic solvent with a halide compound of the formula

Rf(CH₂CH₂X)n (2)

wherein Rf is C_{2∼13} fluoroalkyl or C_{2∼13} fluoroalkylene group, X is iodine or bromine, n is 1 when Rf is fluoroalkyl group and n is 2 when Rf is fluoroalkylene group.

Preferable examples of betaine compounds used in the above method are those having the formula

R¹R²R³N^{⊕}CH₂CO₂^{⊖} (3)

wherein R¹, R² and R³ are each C_{1∼24} alkyl group. Examples thereof are (CH₃)₃N^{⊕}CH₂CO₂^{⊖}, (C₂H₅)₃N^{⊕}CH₂CO₂^{⊖}, C₈H₁₇(CH₃)₂N^{⊕}CH₂CO₂^{⊖}, C₁₂H₂₅(CH₃)₂N^{⊕}CH₂CO₂^{⊖} and C₁₈H₃₇(CH₃)₂N^{⊕}CH₂CO₂^{⊖}.
These betaine compounds are prepared by methods disclosed for example in USP 2,800,502.

The organic solvent used in the above method is preferably soluble with water and promotes a mixing of the starting halide compound and water. Examples of useful organic solvents are methanol, ethanol, propanol or like alcohols, acetone, acetonitrile, ethylene glycol, glycerin, glyme, cellosolve, propionic acid, phenol, cresol, sulfolane, N-methylpyrrolidone, γ-butyrolactone, etc.

In the above method, the betaine compound (3) is used in an amount of usually 1 to 20 equivalents, preferably 1.2 to 5 equivalents per equivalent of the starting halide compound. Further, water is used in an amount of usually 1 to 200 equivalents, preferably 2 to 140 equivalents per equivalent of the starting halide compound, one mole of water being two equivalents. The organic solvent is used in an amount of 0.1 to 10 parts by volume, preferably 1 to 5 parts by volume per parts by volume of water. The reaction temperature is usually 80 to 200°C and preferably 120 to 160°C in view of high reaction velocity and easy heating procedure. The reaction time is usually sufficient in 1 to 10 hours and preferably 2 to 6 hours.

Further, as a second embodiment, the present invention provides a process for preparing a fluoroalcohol of the formula

Rf(CH₂CH₂OH)n (1)

which is a single-step process and comprises contacting a betaine type surfactant and water with a halide compound of the formula

Rf(CH₂CH₂X)n (2)

wherein Rf, X and n are same as above. It is possible, in this method, to omit an organic solvent by using the betaine type surfactant.

Preferable examples of betaine type surfactants used in the above method are those having the formula

R⁴R⁵R⁶N^{⊕}CH₂CO₂^{⊖} (4)

wherein R⁴ and R⁵ are each C_{1∼5} alkyl group and R⁶ is C_{6∼24}, preferably C_{8∼20} alkyl group. Examples thereof are C₈H₁₇(CH₃)₂N^{⊕}CH₂CO₂^{⊖}, C₁₂H₂₅(CH₃)₂N^{⊕}CH₂CO₂^{⊖} and C₁₈H₃₇(CH₃)₂N^{⊕}CH₂CO₂^{⊖}.

Further, in the above method, it is possible, as required, to replace a part or most of the above betaine type surfactant by a betaine compound of the formula

R⁷R⁸R⁹N^{⊕}CH₂CO₂^{⊖} (5)

wherein R⁷, R⁸ and R⁹ are each C_{1∼5} alkyl group. These betaine type surfactants and other betaine compounds are prepared by methods disclosed for example in USP 2,800,502.

In the above method, the betaine type surfactant is used in an amount of usually 1 to 20 equivalents, preferably 1.2 to 5 equivalents per equivalent of the starting halide compound. When the betaine type surfactant is replaced by other betaine compound, the betaine type surfactant is used preferably in an amount of at least 0.2 equivalent. Further, water is used in an amount of usually 1 to 200 equivalents, preferably 2 to 140 equivalents per equivalent of the starting halide compound, one mole of water being two equivalents. The reaction temperature is usually 80 to 200°C and preferably 120 to 160°C in view of high reaction velocity and easy heating procedure. The reaction time is usually sufficient in 1 to 10 hours and preferably 2 to 6 hours. In the above method, since the halide compound is contacted with a betaine type surfactant, an organic solvent needs not to be used.

The present applicant has previously filed a patent application (JP-A-199923/1989) in which the same starting halide compound as in the present invention is contacted with a betaine compound to obtain an intermediate and subsequently subjecting the intermediate to hydrolysis. This method is two-step reaction which comprises a first step of obtaining the intermediate in the absence of water and a second step of hydrolysis in the presence of an aqueous solution of alkali, hence differs from the present single process comprising one-step reaction.

The desired compound of the invention can be separated and purified by known methods, for example, by extraction, distillation, recrystallization, gas chromatography and column chromatography.

The present processes are safe without use of dangerous chemicals and heavy metals. Further, in the present processes, conversion of the starting compound and selectivity of the desired compound are excellent.

The present invention is described by showing examples below.

### Example 1

Into a 200-mℓ autoclave were placed 57.4g (100 mmol) of CF₃(CF₂)₇CH₂CH₂I, 14.1g (120mmol) of (CH₃)₃N^{⊕}CH₂COO^{⊖}, 50g of isopropanol (IPA) and 20g of water, and the mixture was stirred at 150°C for 6 hours. After cooled to room temperature, the reaction mixture was extracted with each 100mℓ of water and trichlorotrifluoroethane (R-113). Analysis of an oil layer by gas chromatography showed CF₃(CF₂)₇CH₂CH₂OH was obtained in 97% conversion and 93% selectivity.

### Example 2

Into a 200-mℓ autoclave were placed 61.0g (100 mmol) of ICH₂CH₂(CF₂)₆CH₂CH₂I, 28.1g (240mmol) of (CH₃)₃N^{⊕}CH₂COO^{⊖}, 60g of IPA and 30g of water, and the mixture was stirred at 150°C for 6 hours. After cooled to room temperature, the reaction mixture was extracted with each 100mℓ of water and R-113. Analysis of an oil layer by gas chromatography showed HOCH₂CH₂(CF₂)₆CH₂CH₂OH was obtained in 87% selectivity. Conversion of ICH₂CH₂(CF₂)₆CH₂CH₂I was 95% and 10% of CH₂=CH(CF₂)₆CH₂CH₂OH was formed as a by-product.

### Example 3

Into a 200-mℓ autoclave wre placed 57.4g (100 mmol) of CF₃(CF₂)₇CH₂CH₂I, 11.7g (100mmol) of (CH₃)₃N^{⊕}CH₂COO^{⊖}, 40g of IPA, 15g of water and 5.4g (20mmol) of C₁₂H₂₅(CH₃)₂N^{⊕}CH₂COO^{⊖} as a betaine type surfactant, and the mixture was stirred at 150°C for 6 hours. After cooled to room temperature, the reaction mixture was extracted with each 100mℓ of water and R-113. Analysis of an oil layer by gas chromatography showed CF₃(CF₂)₇CH₂CH₂OH was obtained in 99% conversion and 95% selectivity.

### Examples 4 to 7

The reactions were conducted in the same manner as in Example 1 except that solvents listed in Table 1 were used in place of isopropanol. Table 1 also shows conversion of CF₃(CF₂)₇CH₂CH₂I and selectivity of CF₃(CF₂)₇CH₂CH₂OH.

**Table 1**

| Ex. | Organic solvent | Conv. | Selec. |
|---|---|---|---|
| 4 | m-cresol | 92% | 98% |
| 5 | ethanol | 100% | 95% |
| 6 | methanol | 97% | 88% |
| 7 | acetonitrile | 96% | 92% |

### Comparison Example 1

The reaction was conducted in the same manner as in Example 1 except that the betaine compound of Example 1 was not used. Conversion of CF₃(CF₂)₇CH₂CH₂I was 0%.

### Example 8

Into a 200-mℓ autoclave were placed 57.4g (100 mmol) of CF₃(CF₂)₇CH₂CH₂I, 100g of water and 32.6g (120mmol) of C₁₂H₂₅(CH₃)₂N^{⊕}CH₂COO^{⊖} as a betaine type surfactant, and the mixture was stirred at 150°C for 6 hours. After cooled to room temperature, the reaction mixture was extracted with each 100mℓ of water and R-113. Analysis of an oil layer by gas chromatography showed CF₃(CF₂)₇CH₂CH₂OH was obtained in 99% conversion and 96% selectivity.

### Example 9

Into a 200-mℓ autoclave were placed 61.0g (100mmol) of ICH₂CH₂(CF₂)₆CH₂CH₂I, 100g of water and 65.1g (240mmol) of C₁₂H₂₅(CH₃)₂N^{⊕}CH₂COO^{⊖} as a betaine type surfactant, and the mixture was stirred at 150°C for 6 hours. After cooled to room temperature, the reaction mixture was extracted with each 100mℓ of water and R-113. Analysis of an oil layer by gas chromatography showed HOCH₂CH₂(CF₂)₆CH₂CH₂OH was obtained in 90% selectivity. Conversion of ICH₂CH₂(CF₂)₆CH₂CH₂I was 99% and 8% of CH₂=CH(CF₂)₆CH₂CH₂OH was formed as a by-product.

### Example 10

Into a 200-mℓ autoclave were placed 43.1g (75 mmol) of CF₃(CF₂)₇CH₂CH₂I, 8.8g (75mmol) of (CH₃)₃N^{⊕}CH₂COO^{⊖}, 60g of water and 4.1g (15mmol) of C₁₂H₂₅(CH₃)₂N^{⊕}CH₂COO^{⊖} as a betaine type surfactant, and the mixture was stirred at 150°C for 6 hours. After cooled to room temperature, the reaction mixture was extracted with each 100mℓ of water and R-113. Analysis of an oil layer by gas chromatography showed CF₃(CF₂)₇CH₂CH₂OH was obtained in 99% conversion and 97% selectivity.

### Comparison Example 2

The reaction was conducted in the same manner as in Example 8 except that 32.8g (120mmol) of sodium laurylsulfate (anionic surfactant) was used in place of the betaine type surfactant of Example 8. Conversion of CF₃(CF₂)₇CH₂CH₂I was 0%.

### Comparison Example 3

The reaction was conducted in the same manner as in Example 8 except that 41.8g (120mmol) of octadecyltrimethylammonium chloride (cationic surfactant) was used in place of the betaine type surfactant of Example 8. Conversion of CF₃(CF₂)₇CH₂CH₂I was 0%.

### Comparison Examole 4

The reaction was conducted in the same manner as in Example 8 except that 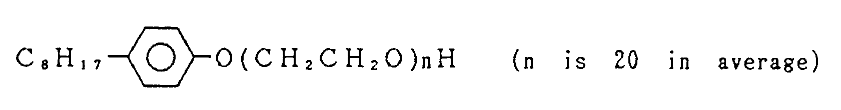
(nonionic surfactant) was used in place of the betaine type surfactant of Example 8. Conversion of CF₃(CF₂)₇CH₂CH₂I was 0%.

## Claims

1. A process for preparing a fluoroalcohol of the formula
Rf(CH₂CH₂OH)n (1)
which comprises contacting a betaine compound and water in the presence of an organic solvent with a halide compound of the formula
Rf(CH₂CH₂X)n (2)
wherein Rf is C_{2∼13} fluoroalkyl or C_{2∼13} fluoroalkylene group, X is iodine or bromine, n is 1 when Rf is fluoroalkyl group and n is 2 when Rf is fluoroalkylene group.

2. A process as defined in claim 1 wherein the betaine compound has the formula
R¹R²R³N^{⊕}CH₂CO₂^{⊖} (3)
wherein R¹, R² and R³ are each C_{1∼24} alkyl group.

3. A process for preparing a fluoroalcohol of the formula
Rf(CH₂CH₂OH)n (1)
which comprises contacting a betaine type surfactant and water with a halide compound of the formula
Rf(CH₂CH₂X)n (2)
wherein Rf, X and n are same as above.

4. A process as defined in claim 3 wherein the betaine type surfactant has the formula
R⁴R⁵R⁶N^{⊕}CH₂CO₂^{⊖} (4)
wherein R⁴ and R⁵ are each C_{1∼5} alkyl group and R⁶ is C_{6∼24} alkyl group.

5. A process as defined in claim 4 wherein a part or most of the betaine type surfactant is replaced by a betaine compound of the formula
R⁷R⁸R⁹N^{⊕}CH₂CO₂^{⊖} (5)
wherein R⁷, R⁸ and R⁹ are each C_{1∼5} alkyl group.

## Patentansprüche

1. Verfahren zur Herstellung eines Fluoralkohols der Formel
Rf(CH₂CH₂OH)ₙ (1)
welches umfaßt das Kontaktieren einer BetainVerbindung und Wasser in Gegenwart eines organischen Lösungsmittels mit einem Halogenid der Formel
Rf(CH₂CH₂X)ₙ (2)
worin Rf eine C₂₋₁₃-Fluoralkyl- oder c₂₋₁₃-Fluoralkylengruppe ist, X Iod oder Brom ist, n 1 ist, wenn Rf eine Fluoralkylgruppe ist, und n 2 ist, wenn Rf eine Fluoralkylengruppe ist.

2. Verfahren nach Anspruch 1, worin die Betain-Verbindung die Formel
R¹R²R³N⁺CH₂CO₂⁻ (3)
hat, worin R¹, R² und R³ jeweils eine C₁₋₂₄-Alkylgruppe sind.

3. Verfahren zur Herstellung eines Fluoralkohols der Formel
Rf(CH₂CH₂OH)ₙ (1)
welches umfaßt das Kontaktieren eines Tensids vom Betain-Typ und Wasser mit einem Halogenid der Formel
Rf(CH₂CH₂X)ₙ (2)
worin Rf, X und n wie oben sind.

4. Verfahren nach Anspruch 3, worin das Tensid von Betain-Typ die Formel
R⁴R⁵R⁶N⁺CH₂CO₂⁻ (4)
hat, worin R⁴ und R⁵ jeweils eine C₁₋₅-Alkylgruppe und R⁶ eine C₆₋₂₄-Alkylgruppe sind.

5. Verfahren nach Anspruch 4, worin ein Teil oder das meiste Tensid vom Betain-Typ durch eine BetainVerbindung der Formel
R⁷R⁸R⁹N⁺CH₂CO₂⁻ (5)
ersetzt ist, worin R⁷, R⁸ und R⁹ jeweils eine C₁₋₅-Alkylgruppe sind.

## Revendications

1. Procédé pour préparer un fluoroalcool représenté par la formule :
Rf(CH₂CH₂OH)ₙ (1)
qui comprend la mise en contact d'un composé de type bétaïne et d'eau en présence d'un solvant organique avec un halogénure représenté par la formule:
Rf(CH₂CH₂X)n (2)
dans lesquelles Rf est un groupe fluoroalkyle en C_{2∼13} ou fluoroalkylène en C_{2∼13}, X est un atome d'iode ou de brome, n est 1 lorsque Rf est un groupe fluoroalkyle et n est 2 lorsque Rf est un groupe fluoroalkylène.

2. Procédé suivant la revendication 1, dans lequel le composé de type bétaine est représenté par la formule :
R¹R²R³N⁺ CH₂CO₂⁻ (3)
dans laquelle R¹, R² et R³ sont chacun un groupe alkyle en C_{2∼24}.

3. Procédé pour préparer un fluoroalcool représenté par la formule :
Rf(CH₂CH₂OH)ₙ (1)
qui comprend la mise en contact d'un agent tensioactif de type bétaïne et d'eau avec un halogénure représenté par la formule:
Rf(CH₂CH₂X)ₙ (2)
dans lesquelles Rf, X et n sont tels que définis plus haut.

4. Procédé suivant la revendication 3, dans lequel l'agent tensioactif de type bétaine est représenté par la formule :
R⁴R⁵R⁶N⁺ CH₂CO₂⁻ (4)
dans laquelle R⁴ et R⁵ sont chacun un groupe alkyle en C_{1∼5} et R⁶ est un groupe alkyle en C_{6∼24}.

5. Procédé suivant la revendication 4, dans lequel une partie ou la plus grande partie de l'agent tensioactif de type bétaine est remplacée par un composé de type bétaïne représenté par la formule :
R⁷R⁸R⁹N⁺ CH₂CO₂⁻ (5)
dans laquelle R⁷, R⁸ et R⁹ sont chacun un groupe alkyle en C_{1∼5}.
